Europäisches Patentamt

European Patent Office

Office européen des brevets

(19)

(11) Veröffentlichungsnummer: **0 272 378 B1**

# EUROPÄISCHE PATENTSCHRIFT

(12)

(45) Veröffentlichungstag der Patentschrift: **18.09.91**

(51) Int. Cl.⁵: **C07C  11/04, //F22B1/18**

(21) Anmeldenummer: **87112640.5**

(22) Anmeldetag: **29.08.87**

(54) Verfahren und Vorrichtung zum Kühlen von Spaltgas.

(30) Priorität: 20.12.86 DE 3643801

(43) Veröffentlichungstag der Anmeldung:
29.06.88 Patentblatt  88/26

(45) Bekanntmachung des Hinweises auf die
Patenterteilung:
18.09.91 Patentblatt  91/38

(84) Benannte Vertragsstaaten:
DE FR GB IT NL

(56) Entgegenhaltungen:
EP-A- 0 062 344
WO-A-84/00059
FR-A- 1 469 918

(73) Patentinhaber: **Deutsche Babcock-Borsig AG**
**Berlinerstrasse 27-37**
**W-1000 Berlin 27(DE)**

(72) Erfinder: **Kehrer, Wolfgang**
**Peter-Strasser-Weg 18**
**W-1000 Berlin 41(DE)**

(74) Vertreter: **Müller, Jürgen, Dipl.-Ing.**
**Deutsche Babcock AG Lizenz- und Patentab-**
**teilung Duisburger Strasse 375**
**W-4200 Oberhausen 1(DE)**

**Beschreibung**

Die Erfindung betrifft ein Verfahren und eine Vorrichtung zum Kühlen von Spaltgas in einer Äthylenanlage nach dem Oberbegriff der Ansprüche 1 und 4.

Das durch eine thermische Spaltung von Kohlenwasserstoffen erzeugte Spaltgas ist ein Gemisch aus Kohlenwasserstoffen unterschiedlichen Molekulargewichts und Partialdrucks. Dieses Spaltgas muß zur Stabilisierung seiner molekularen Zusammensetzung sehr schnell abgekühlt werden, was durch indirekte Wärmeübertragung von dem Spaltgas an das als wärmeaufnehmendes Medium dienende verdampfende Wasser in Spaltgaskühlern geschieht. Die Spaltgaskühler bestehen aus von dem Spaltgas durchströmten Rohren, die auf der Mantelseite von dem wärmeaufnehmenden Medium umgeben sind. Durch das verdampfende Wasser wird eine intensive Kühlung der Rohre erzielt, wodurch die Rohrwandtemperatur relativ niedrig ist und nur wenig über der Temperatur des verdampfenden Wassers liegt.

In der FR-A-1 469 918 ist ein Verfahren zur Erzeugung von Äthylen durch eine thermische Spaltung von Kohlenwasserstoffen in einem Spaltgasofen und durch eine anschließende zweistufige Kühlung beschrieben. Die erste Kühlstufe ist durch einen unmittelbar an den Spaltgasofen angeschlossenen Einrohrkühler gebildet, an den sich als zweite Kühlstufe ein Mehrrohrkühler anschließt. Auf diese Weise soll die Verweilzeit des Spaltgases in dem nicht beheizten und nicht gekühlten Übergangsbereich zwischen dem Spaltgasofen und den Spaltgaskühlern extrem gering gehalten werden. Durch diese kurze Verweilzeit wird eine Rückreaktion der Spaltgaskomponenten vermieden, die zu einer Bildung von Kohlenstoff in dem Übergangsbereich führen kann. Als Kühlmedium wird auch bei diesem Verfahren verdampfendes Wasser verwendet, das den beiden Kühlstufen über getrennte Anschlüsse zugeführt wird.

Während der Abkühlung des Spaltgases in dem Spaltgaskühler kann für einige Komponenten des Spaltgases die Kondensationstemperatur unterschritten werden. Als Folge davon kommt es im Bereich der niedrigen Temperaturen zur Ausscheidung dieser Komponenten an der Rohrwand und damit zu dem Aufbau einer sogenannten Koksschicht. Diese Koksschicht erhöht den Strömungswiderstand, wodurch sich der Gasdruck in dem vorgeschalteten Spaltofen erhöht. Schlechtere Spaltgasausbeute, weitere Erhöhung der Koksschichtdicke, steigende Gasaustrittstemperaturen und geringere Dampferzeugung sind die Folge. Nach einer gewissen Betriebszeit muß daher der Spaltgaskühler zur Beseitigung der Koksschicht außer Betrieb gesetzt werden.

Eine aus der DE-A-2 331 686 bekannte Vorrichtung zur Kühlung von Spaltgas ist ein Röhrenwärmetauscher stehender Bauart, in dessen unterem Teil ein Wasserbad und in dessen oberem Teil ein Dampfraum vorhanden ist, in den der Wasserdampf aus dem Wasserbad unmittelbar übertritt. In diesem Wärmetauscher wird das Spaltgas in drei Stufen gekühlt, wobei die Kühlung in der ersten und der dritten Stufe durch verdampfendes Wasser und in der zweiten Stufe durch den entstandenen Wasserdampf erfolgt. Diese Vorrichtung ist auf die Erzeugung von überhitztem Dampf ausgelegt, ohne daß die Einhaltung bestimmter Rohrwandtemperaturen zur Vermeidung der Auskondensation von schweren Kohlenwasserstoffen berücksichtigt ist.

In der EP-A-0 062 344 ist ein System zur Erzeugung von überhitztem Dampf unter Ausnutzung der Wärme eines in einer Ammoniaksyntheseanlage hergestellten Gases beschrieben. Dieses heiße Gas wird zuerst einem Abhitzekessel zur Verdampfung von Wasser und anschließend einem davon getrennten Wärmetauscher zur Überhitzung des erzeugten Dampfes zugeführt.

Der Erfindung liegt die Aufgabe zugrunde, bei einem Verfahren zur Äthylenherstellung den Kühlprozeß des Spaltgases so zu führen, daß keine Kohlenwasserstoffe auf Grund einer zu niedrigen Rohrtemperatur auskondensieren können.

Diese Aufgabe wird bei einem gattungsgemäßen Verfahren erfindungsgemäß durch die kennzeichnenden Merkmale des Anspruches 1 gelöst. Eine vorteilhafte Ausgestaltung des erfindungsgemäßen Verfahrens ist in den Ansprüchen 2 und 3, und eine Vorrichtung zur Durchführung des Verfahrens ist in den Ansprüchen 4 und 5 angegeben.

Das erfindungsgemäße Verfahren berücksichtigt, daß die Höhe der Oberflächentemperatur der mit dem Spaltgas in Berührung stehenden Wärmeaustauschfläche die Verkokung und damit die Laufzeit und die Dampferzeugung des Wärmetauschers sowie die Spaltgasausbeute maßgeblich beeinflußt. Wie bei dem bekannten Verfahren wird daher das Spaltgas zunächst durch verdampfendes Wasser rasch abgekühlt. Jedoch wird dann in dem gesamten hinteren, d.h. in dem kälteren Teil des Wärmetauschers, wo bisher starke Verkokungen auftraten, das Spaltgas ausschließlich durch Dampf gekühlt. Der Dampf hat eine im Vergleich zu siedendem Wasser geringere Wärmeübertragungszahl. Infolge dessen nehmen die Rohre bis zum Gasaustritt eine höhere Wandtemperatur an, wodurch weniger Kohlenwasserstoffe kondensieren und die Koksbildung vermindert wird.

Zwei Ausführungsbeispiele der Erfindung sind in der Zeichnung dargestellt und werden im folgenden näher erläutert. Es zeigen:

Fig. 1    schematisch den Längsschnitt durch eine Vorrichtung gemäß der Erfindung und

Fig. 2   schematisch den Längsschnitt durch eine andere Vorrichtung gemäß der Erfindung.

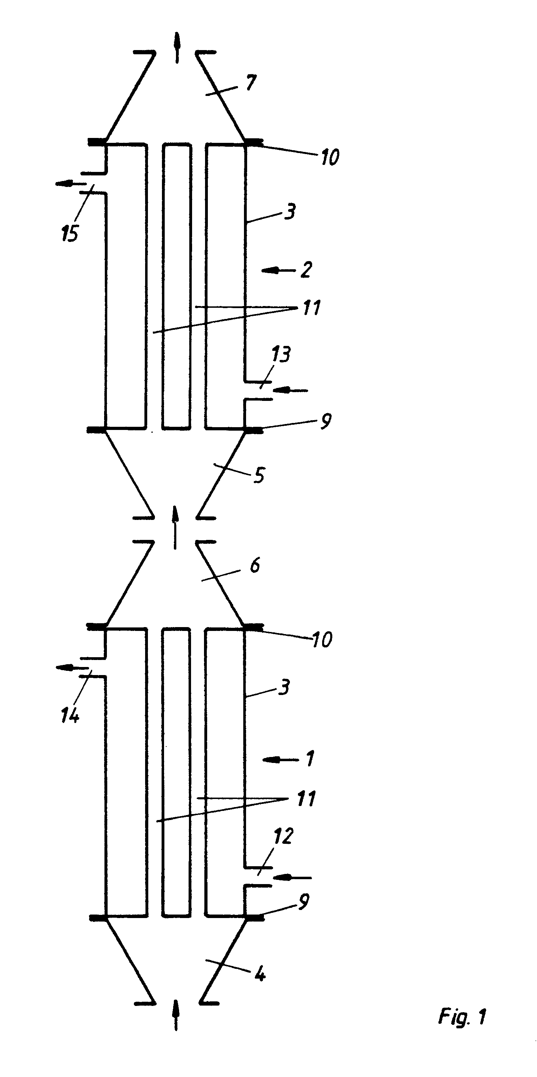

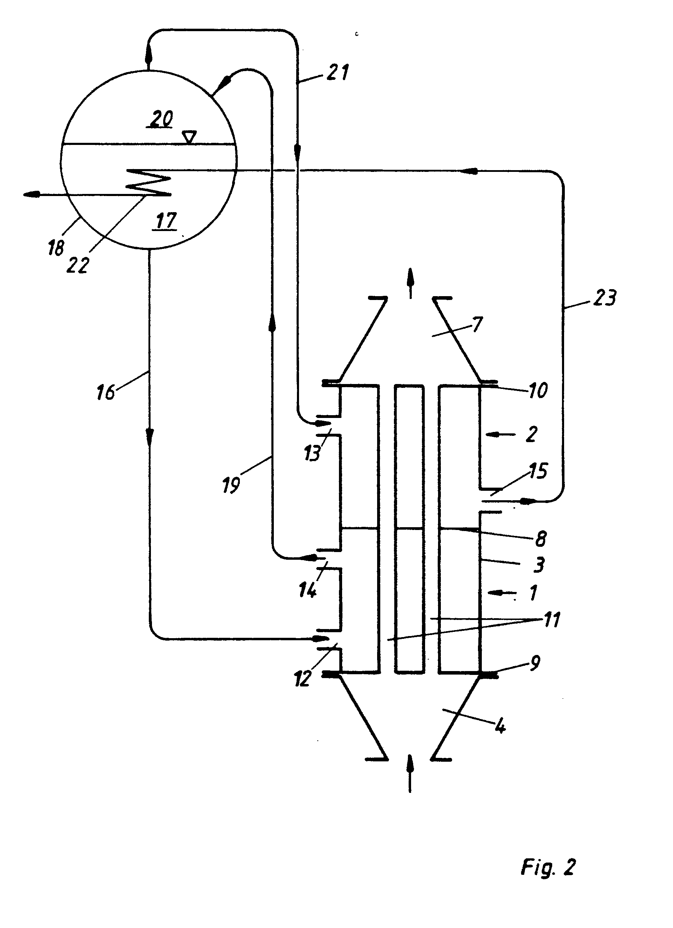

Die dargestellte Vorrichtung ist innerhalb einer Äthylenanlage angeordnet und umfaßt zwei Kühlstufen 1, 2, die gemäß Fig. 1 aus zwei voneinander getrennten Röhrenwärmetauschern bestehen. Die Kühlstufen 1, 2 können auch gemäß Fig. 2 zu einem Röhrenwärmetauscher zusammengefaßt werden. Jeder Röhrenwärmetauscher enthält einen äußeren Mantel 3, der mit einer Gaseintrittskammer 4, 5 und einer Gasaustrittskammer 6, 7 versehen ist. Die Gaseintrittskammer 4 der ersten Kühlstufe ist mit einem Spaltgasofen verbunden.

Die Gasaustrittskammer 7 der zweiten Kühlstufe 2 ist an eine Leitung zur Weiterleitung des Gases angeschlossen. Die genannte Leitung und der Spaltgasofen sind aus Gründen der Übersichtlichkeit nicht dargestellt. Die Gasaustrittskammer 6 der ersten Kühlstufe 1 ist mit der Gaseintrittskammer 5 der zweiten Kühlstufe 2 verbunden, so daß beide Kühlstufen 1, 2 von dem Spaltgas nacheinander durchströmt sind. Sind gemäß Fig. 2 beide Kühlstufen 1, 2 innerhalb eines Mantels 3 angeordnet, so sind die beiden Kühlstufen 1, 2 durch eine Trennwand 8 voneinander getrennt.

Jeder Röhrenwärmetauscher enthält zwei Rohrböden 9, 10, die mit dem Mantel 3 dicht verbunden sind. In den Rohrböden 9, 10 sind gerade, von dem Spaltgas durchströmte Rohre 11 gehalten. Bei dem Wärmetauscher gemäß Fig. 2 durchdringen die Rohre 11 die Trennwand 8. Der Mantel 3 einer jeden Kühlstufe 1, 2 ist jeweils mit einem Eintrittsstutzen 12, 13 und einem Austrittsstutzen 14, 15 für ein wärmeaufnehmendes Medium versehen, das den Raum zwischen dem Mantel 3 und den Rohrböden 9, 10 durchströmt.

Der ersten Kühlstufe 1 wird durch den Eintrittsstutzen 12 Wasser zugeführt, das durch den Austrittsstutzen 14 als Wasser-Dampf-Gemisch die Kühlstufe 1 verläßt. Zu diesem Zweck kann der Eintrittsstutzen 12 über eine Falleitung 16 mit dem Wasserraum 17 einer Dampftrommel 18 verbunden sein, die der Trennung von Wasser und Dampf dient. Der Austrittsstutzen 14 der ersten Kühlstufe 1 ist über eine Steigleitung 19 mit dem Dampfraum 20 der Dampftrommel 18 verbunden. Der zweiten Kühlstufe 2 wird über den Eintrittsstutzen 13 Dampf zugeführt, der durch Wärmeaufnahme überhitzt wird und durch den Austrittsstutzen 15 abgeführt wird. Der Eintrittsstutzen 13 ist über eine Leitung 21 mit dem Dampfraum 20 der Dampftrommel 18 verbunden. In dem Wasserraum 17 der Dampftrommel 18 ist eine Rohrschlange 22 angeordnet. Diese Rohrschlange 22 ist über eine Leitung 23 mit dem Austrittsstutzen 15 der zweiten Kühlstufe 2 verbunden.

Das in dem Spaltofen erzeugte Spaltgas tritt in die Gaseintrittskammer 4 der ersten Kühlstufe 1 ein und wird im Wärmetausch mit dem verdampfenden Wasser rasch gekühlt, wobei die Gastemperatur am Ende der ersten Kühlstufe 600 Grad C betragen kann. Das Spaltgas wird anschließend in der zweiten Kühlstufe 2 auf die Endtemperatur mit Hilfe von Dampf gekühlt und mit dieser Temperatur der Weiterverarbeitungseinrichtung der Äthylenanlage zugeführt. In der zweiten Kühlstufe 2 läßt sich durch den Wärmetausch mit dem Dampf eine Wandtemperatur der Rohre einhalten, die hoch genug ist, daß sich schwere Kohlenwasserstoffe an keiner Stelle innerhalb der Rohre 11 abscheiden können. Der für die zweite Kühlstufe 2 verwendete Dampf kann aus dem die erste Kühlstufe 1 verlassenden Wasser-Dampf-Gemisch durch eine Trennung in der Dampftrommel 18 gewonnen werden.

## Patentansprüche

1. Verfahren zum indirekten Kühlen von Spaltgas in einer Äthylenanlage, bei der das Spaltgas in zwei Stufen auf die vorgesehene Endtemperatur gekühlt wird und in der ersten Stufe verdampfendes Wasser als Kühlmittel verwendet wird, dadurch **gekennzeichnet,** daß in der zweiten Stufe das Spaltgas ausschließlich durch zugeführten Dampf gekühlt wird.

2. Verfahren nach Anspruch 1, dadurch **gekennzeichnet,** daß das die erste Kühlstufe verlassende Wasser-Dampf-Gemisch von dem Wasseranteil befreit wird und daß der Dampfanteil der zweiten Kühlstufe zugeführt wird.

3. Verfahren nach Anspruch 1 und 2, dadurch **gekennzeichnet,** daß das Spaltgas in der ersten Kühlstufe auf 600° C gekühlt wird.

4. Vorrichtung zum indirekten Kühlen von Spaltgas in einer Äthylenanlage, bei der von dem Spaltgas durchströmte und von einem Mantel (3) umgebene Rohre (11) in zwei Kühlstufen angeordnet sind, die durch zwei von dem Mantel (3) umschlossene, voneinander getrennte, jeweils mit eigenen Anschlüssen (12, 13, 14, 15) für die Zufuhr und die Abfuhr von Wasser oder Dampf versehene und an eine einen Wasserraum (17) und einen Dampfraum (20) aufweisende Dampftrommel (18) angeschlossene Räume gebildet sind, wobei in der ersten Kühlstufe (1) der Anschluß (12) für die Zufuhr von Wasser mit dem Wasserraum (17) und der Anschluß für die Abfuhr von Dampf (14) mit dem Dampfraum (20) der Dampftrommel (18) verbunden ist und die Rohre (11) hinter der zweiten Kühlstufe (2), in eine mit einer Weiterverarbeitungseinrichtung verbundene Gasaustrittskammer (7) münden, dadurch **gekennzeichnet,** daß der Zufuhranschluß (13) der zweiten Kühlstufe (2) mit dem Dampfraum (20) der Dampftrommel (18) verbunden ist.

5. Vorrichtung nach Anspruch 4 und 5, dadurch **gekennzeichnet,** daß die die Kühlstufen (1, 2) bildenden Räume in voneinander getrennten und gasseitig miteinander verbundenen Wärmetauschern angeordnet sind.

## Claims

1. Method for the indirect cooling of cracking gas in an ethylene plant, in which the cracking gas is cooled in two stages to the envisaged final temperature and evaporating water is used as coolant in the first stage, characterised thereby, that the cracking gas is cooled in the second stage exclusively by supplied steam.

2. Method according to claim 1, characterised thereby, that the mixture of water and steam leaving the first cooling stage is freed of the water component and that the steam component is fed to the second cooling stage.

3. Method according to claim 1 and 2, characterised thereby, that the cracking gas is cooled to 600°C in the first cooling stage.

4. Device for the indirect cooling of cracking gas in an ethylene plant, in which tubes (11), which are surrounded by a jacket (3) and flowed through by cracking gas, are arranged in two cooling stages which are formed by two chambers, which are enclosed by the jacket (3), are each separated from the other, are each provided with their own connections (12, 13, 14, 15) for the feed and removal of water or steam and which are connected to a steam drum (18) displaying a water chamber (17) and a steam chamber (20), wherein the connection (12) for the feed of water in the first cooling stage (1) is connected with the water chamber (17) and the connection (14) for the removal of steam in the first cooling stage (1) is connected with the steam chamber (20) of the steam drum (18) and the tubes (11) open out behind the second cooling stage (2) into a gas exit chamber (7) connected with a further processing equipment, characterised thereby, that the feed connection (13) of the second cooling stage (2) is connected with the steam chamber (20) of the steam drum (18).

5. Device according to claim 4 and 5, characterised thereby, that the chambers forming the cooling stages (1, 2) are arranged in heat exchangers which are each separated from the other and each connected with the other on the gas side.

## Revendications

1. Procédé de refroidissement indirect de gaz de crackage dans une installation d'éthylène, dans laquelle le gaz de crackage est refroidi en deux étapes à la température finale prévue et de l'eau étant utilisée comme fluide de refroidissement dans le premier étage, caractérisé en ce que le gaz de crackage est refroidi dans la deuxième étape exclusivement par une amenée de vapeur.

2. Procédé selon la revendication 1, caractérisé en ce que le mélange eau-vapeur qui quitte le premier étage de refroidissement est purgé de la partie d'eau et que la partie de vapeur est amenée au deuxième étage de refroidissement.

3. Procédé selon les revendications 1 et 2, caractérisé en ce que le gaz de crackage est refroidi à 600°C dans la première étape de refroidissement.

4. Dispositif pour le refroidissement indirect de gaz de crackage dans une installation d'éthylène, dans lequel des tubes (11) parcourus par le gaz de crackage et entourés par une enveloppe (3) sont disposés en deux étages de refroidissement, formés par deux enceintes, entourées par l'enveloppe (3), séparées l'une de l'autre, pourvues chacune de raccordements (12,13,14,15) propres, pour l'amenée et l'évacuation d'eau ou de vapeur, et raccordées à un tambour de vapeur (18) présentant une enceinte d'eau (17) et une enceinte de vapeur (20), où, dans le premier étage, le raccordement (12) pour d'amenée d'eau est relié à l'enceinte d'eau (17) et le raccordement pour l'évacuation de vapeur (14) est relié à l'enceinte de vapeur (20) du tambour de vapeur (18), et les tubes (11) débouchant derrière le deuxième étage de refroidissement (2), dans une chambre de sortie de gaz (7) reliée à un dispositif de retraitement, caractérisé en ce que le raccordement d'amenée de vapeur (13) du deuxième étage de refroidissement (2) est relié à l'enceinte de vapeur (20) du tambour de vapeur (18).

5. Dispositif selon les revendications 4 et 5, caractérisé en ce que les enceintes qui forment les étages de refroidissement (1,2) sont disposées dans des échangeurs de chaleur séparés l'un de l'autre et reliés entre eux côté gaz.

*Fig. 1*

*Fig. 2*